(19) Europäisches Patentamt / European Patent Office / Office européen des brevets

(11) **EP 3 233 991 B1**

(12) **EUROPEAN PATENT SPECIFICATION**

(45) Date of publication and mention of the grant of the patent:
**01.02.2023 Bulletin 2023/05**

(21) Application number: **15823568.9**

(22) Date of filing: **16.12.2015**

(51) International Patent Classification (IPC):
**C08J 7/12** *(2006.01)*    **C12M 1/00** *(2006.01)*

(52) Cooperative Patent Classification (CPC):
**C08J 7/123; C12M 23/20;** C08J 2323/12

(86) International application number:
**PCT/US2015/066169**

(87) International publication number:
**WO 2016/100557 (23.06.2016 Gazette 2016/25)**

(54) **PLASMA TREATMENT WITH NON-POLYMERIZING COMPOUNDS THAT LEADS TO REDUCED BIOMOLECULE ADHESION TO THERMOPLASTIC ARTICLES**

PLASMABEHANDLUNG MIT NICHTPOLYMERISIERENDEN VERBINDUNGEN, DIE ZU EINER VERRINGERTEN HAFTUNG VON BIOMOLEKÜLEN AN THERMOPLASTISCHEN GEGENSTÄNDEN FÜHRT

TRAITEMENT AU PLASMA AVEC DES COMPOSÉS NON POLYMÉRISANTS QUI CONDUIT À LA RÉDUCTION DE L'ADHÉRENCE DE BIOMOLÉCULES À DES ARTICLES THERMOPLASTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **17.12.2014  US 201462093194 P**
       **10.02.2015  US 201562114494 P**
       **30.04.2015  US 201562155090 P**
       **19.10.2015  US 201562243392 P**

(43) Date of publication of application:
**25.10.2017 Bulletin 2017/43**

(73) Proprietor: **Si02 Medical Products, Inc.**
**Auburn, AL 36832 (US)**

(72) Inventors:
• **TAHA, Ahmad**
**Auburn, Alabama 36830 (US)**
• **FELTS, John T.**
**Alameda, California 94501 (US)**

(74) Representative: **Maiwald GmbH**
**Elisenhof**
**Elisenstraße 3**
**80335 München (DE)**

(56) References cited:
EP-A1- 1 689 216        WO-A1-96/23834
WO-A1-2007/132018       WO-A1-2009/149827
US-A- 4 445 991         US-A- 5 147 678
US-A- 6 074 871         US-A1- 2005 164 009
US-A1- 2009 291 258     US-A1- 2013 022 752
US-A1- 2014 274 825

**Description**

FIELD OF INVENTION

The invention relates generally to treating a surface, or surface modification of a plastic substrate, to reduce biomolecule adhesion to the surface. More particularly, the invention relates to plasma treatment or surface modification of a plastic substrate, e.g., a medical device or item of laboratory ware, using non-polymerizing compounds to reduce protein adhesion to the substrate surface.

BACKGROUND

In blood, biomolecule, and blood analyte testing, it is desirable to minimize biomolecule adsorption and binding to plastic ware used with these biological substances. Plastic microwell plates, chromatography vials, and other containers, as well as pipettes (sometimes spelled "pipets"), pipette tips, centrifuge tubes, microscope slides, and other types of laboratory ware (also known as labware) used to prepare and transfer samples commonly have hydrophobic surfaces and readily adsorb biomolecules such as proteins, DNA, and RNA. Surfaces of these and other types of laboratory ware components made of polymeric plastic can cause binding of the biomolecule samples. It is thus a desire to provide surfaces for plastic laboratory ware and other articles that contact biological substances, to reduce a wide range of biomolecules from adhering.

SUMMARY

Accordingly, the invention is directed to a method for treating a surface of a plastic substrate including at least two treatment steps, as defined in the attached claims. The first treatment step includes treating the surface with remote conditioning plasma of one or more non-polymerizing compounds. The second treatment step includes treating the surface with remote conversion plasma of water to form a converted surface. The converted surface has a biomolecule recovery percentage greater than the biomolecule recovery percentage of the surface prior to treatment according to the method.

The invention is also directed to an article containing a plastic substrate with a surface treated according to the method according to the present invention.

BRIEF DESCRIPTION OF THE DRAWINGS

The invention will be described in conjunction with the following drawings in which like reference numerals designate like elements and wherein:

Fig.1 illustrates generically described remote conversion plasma treatment apparatus useful in the invention, certain features of which are optional.

Fig. 2 illustrates an exemplary plasma reactor configuration for carrying out remote conversion plasma treatment of microplates according to the invention.

Fig. 3 is a bar graph illustrating comparative biomolecule recovery results between untreated polypropylene microplates, Eppendorf brand microplates and microplates treated with an exemplary remote conversion plasma treatment process according to the invention.

Fig. 4 is a bar graph illustrating comparative biomolecule recovery results between microplates treated with an exemplary remote conversion plasma treatment process according to the invention and microplates treated with the same process steps and conditions except using direct conversion plasma treatment instead of remote conversion plasma treatment.

Fig. 5 is a bar graph illustrating comparative biomolecule recovery results between microplates treated with an exemplary remote conversion plasma treatment process according to the invention and microplates treated with only the non-polymerizing compound step and without the second step.

Fig. 6 illustrates an exemplary radio-frequency-excited plasma reactor configuration according to Fig. 1 for carrying out remote conversion plasma treatment of microplates according to the invention.

Fig. 7 illustrates another exemplary plasma reactor configuration according to Fig. 1 for carrying out remote conversion plasma treatment of microplates according to the invention.

Fig. 8 is an exemplary microwave-excited plasma reactor configuration according to Fig. 1 for carrying out remote conversion plasma treatment of microplates according to the invention.

Fig. 9 is a plot of biomolecule (TFN) recovery from untreated polypropylene beakers, treated polypropylene beakers according to the invention, and glass beakers.

Fig. 10 shows an exemplary reactor configuration for carrying out the method of the present invention. Another suitable reactor configuration is that of Figure 2 as shown and described in U.S. Pat. No. 7,985,188.

Fig. 11 is a plot of protein recovery versus concentration of protein (BSA) for Example 6.

Fig. 12 is a plot of protein recovery versus concentration of protein (PrA) for Example 6.

Fig. 13 is a plot of protein recovery versus concentration of protein (PrG) for Example 6.

The following reference characters are used in this description and the accompanying Figures:

| 9 | Apparatus of the first more detailed embodiment | 36 | Treated polypropylene plot |
|---|---|---|---|
| 10 | Treatment volume | 38 | Glass plot |
| 11 | Reaction chamber wall (optional) | 110 | Chamber |
| 12 | Fluid source | 112 | Bottom (of 110) |
| 13 | Fluid inlet | 114 | Lid (of 110) |
| 14 | Substrate | 116 | Vacuum conduit |
| 15 | Plasma zone | 118 | Vacuum pump |
| 16 | Shield (optional) | 120 | Valve |
| 17 | Treatment gas | 122 | Gas inlet |
| 18 | Plasma energy source | 124 | Processing area |
| 19 | Lid (optional) | 126 | Gas system |
| 20 | Plasma (boundary) | 128 | Mass flow controller |
| 21 | Substrate support (optional) | 130 | Compressed gas source |
| 22 | Vacuum source (optional) | 132 | Capillary |
| 23 | Applicator | 134 | Manifold |
| 24 | Remote conversion plasma | 136 | Shut-off valve |
| 26 | Flat spot (of 20) (optional) | 138 | Electrode |
| 28 | Front surface (of 14) | 140 | Matching network |
| 30 | Back surface (of 14) | 142 | RF power supply |
| 32 | Well (of 14) (optional) | 144 | Coaxial cable |
| 34 | Untreated polypropylene plot | | |

DETAILED DESCRIPTION OF THE PREFERRED EMBODIMENTS

According to the invention, methods are disclosed for reducing biomolecule adhesion to a surface. A method for treating a surface of a plastic substrate, optionally an entire or partial surface of a plastic substrate, is provided, which method is defined in the attached claims.

The term "biomolecule" is used respecting any embodiment to include any nucleotides or peptides, or any combination of them. Nucleotides include oligonucleotides and polynucleotides, also known as nucleic acids, for example deoxyribonucleic acid (DNA) and ribonucleic acid (RNA). Peptides include amino acids, oligopeptides, polypeptides, and proteins. Nucleotides and peptides further include modified or derivatized nucleotides and peptides that adhere to a surface that is not treated according to the present invention.

The presently defined biomolecules include one or more of the following aqueous proteins: mammal serum albumin, for example Bovine Serum Albumin (BSA); Fibrinogen (FBG); Transferrin (TFN), for example blood serotransferrin (or siderophilin, also known as transferrin); lactotransferrin (lactoferrin); milk transferrin; egg white ovotransferrin (conalbumin); membrane-associated melanotransferrin; Protein A (PrA); Protein G (PrG); Protein A/G; Protein L; Insulin, for example hexameric insulin, monomeric insulin, porcine insulin, human insulin, recombinant insulin and pharmaceutical grades of insulin; pharmaceutical protein; blood or blood component proteins; or any recombinant form, modification, full length precursor, signal peptide, pro-peptide, or mature variant of these proteins; and a combination of two or more of these.

Biomolecule adhesion to a surface is defined for any embodiment as a reduction of the aqueous concentration of a biomolecule dispersed in an aqueous medium stored in contact with the surface. It is not limited by the mechanism of reduction of concentration, whether literally "adhesion," adsorption, or another mechanism.

"Plasma," as referenced in any embodiment, has its conventional meaning in physics of one of the four fundamental states of matter, characterized by extensive ionization of its constituent particles, a generally gaseous form, and incandescence (i.e. it produces a glow discharge, meaning that it emits light).

"Conversion plasma treatment" refers to any plasma treatment that reduces the adhesion of one or more biomolecules to a treated surface.

"Conditioning plasma treatment" refers to any plasma treatment of a surface to prepare the surface for further conversion plasma treatment. "Conditioning plasma treatment" includes a plasma treatment that, in itself, reduces the adhesion of one or more biomolecules to a treated surface, but is followed by conversion plasma treatment that further reduces the adhesion of one or more biomolecules to a treated surface. "Conditioning plasma treatment" also includes a plasma treatment that, in itself, does not reduce the adhesion of one or more biomolecules to a treated surface.

A "remote" conversion plasma treatment, generally speaking, is conversion plasma treatment of a surface located at a "remote" point where the radiant energy density of the plasma, for example in Joules per $cm^3$, is substantially less than the maximum radiant energy density at any point of the plasma glow discharge (referred to below as the "brightest point"), but the remote surface is close enough to some part of the glow discharge to reduce the adhesion of one or more biomolecules to the treated remote surface. "Remote" is defined in the same manner respecting a remote conditioning plasma treatment, except that the remote surface must be close enough to some part of the glow discharge to condition the surface.

The radiant energy density at the brightest point of the plasma is determined spectrophotometrically by measuring the radiant intensity of the most intense emission line of light in the visible spectrum (380 nanometer (nm) to 750 nm wavelength) at the brightest point. The radiant energy density at the remote point is determined spectrophotometrically by measuring the radiant energy density of the same emission line of light at the remote point. "Remoteness" of a point is quantified by measuring the ratio of the radiant energy density at the remote point to the radiant energy density at the brightest point. The present specification and claims define "remote" quantitatively as a specific range of that ratio. The ratio is less than 0.5, alternatively less than 0.25, alternatively about 0, alternatively exactly 0. Remote conversion plasma treatment can be carried out where the ratio is zero, even though that indicates no measurable visible light at the remote point, because the dark discharge region or afterglow region of plasma contain energetic species that, although not energetic enough to emit light, are energetic enough to modify the treated surface to reduce the adhesion of one or more biomolecules.

A "non-polymerizing compound" is defined operationally for all embodiments as a compound that does not polymerize on a treated surface or otherwise form an additive coating under the conditions used in a particular plasma treatment of the surface. Numerous examples of compounds that can be used under non-polymerizing conditions are the following: $O_2$, $N_2$, air, $O_3$, $N_2O$, $H_2$, $H_2O_2$, $NH_3$, Ar, He, Ne, and combinations of any of two or more of the foregoing. These may also include alcohols, organic acids, and polar organic solvents as well as materials that can be polymerized under different plasma conditions from those employed. "Non-polymerizing" includes compounds that react with and bond to a preexisting polymeric surface and locally modify its composition at the surface. The essential characterizing feature of a non-polymerizing coating is that it does not build up thickness (i.e. build up an additive coating) as the treatment time is increased.

A "substrate" is an article or other solid form (such as a granule, bead, or particle).

A "surface" is broadly defined as either an original surface (a "surface" also includes a portion of a surface wherever used in this specification) of a substrate, or a coated or treated surface prepared by any suitable coating or treating method, such as liquid application, condensation from a gas, or chemical vapor deposition, including plasma enhanced chemical vapor deposition carried out under conditions effective to form a coating on the substrate.

A converted surface is defined for all embodiments as a surface that has been plasma treated as described in this specification, and that exhibits reduced adhesion by biomolecules as a result of such treatment.

The "material" in any embodiment can be any material of which a plastic substrate is formed, including a thermoplastic material, optionally a thermoplastic injection moldable material. The substrate according to any embodiment may be made, for example, from material including: an olefin polymer; polypropylene (PP); polyethylene (PE); cyclic olefin copolymer (COC); cyclic olefin polymer (COP); polymethylpentene; polyester; polyethylene terephthalate; polyethylene naphthalate; polybutylene terephthalate (PBT); PVdC (polyvinylidene chloride); polyvinyl chloride (PVC); polycarbonate; polymethylmethacrylate; polylactic acid; polylactic acid; polystyrene; hydrogenated polystyrene; poly(cyclohexylethylene) (PCHE); epoxy resin; nylon; polyurethane polyacrylonitrile; polyacrylonitrile (PAN); or an ionomeric resin.

The term "vessel" as used throughout this specification may be any type of article that is adapted to contain or convey a liquid, a gas, a solid, or any two or more of these. One example of a vessel is an article with at least one opening (e.g., one, two or more, depending on the application) and a wall defining an interior contacting surface.

The present method for treating a surface of a plastic substrate includes treating the surface with conditioning plasma of one or more non-polymerizing compounds, in a chamber, to form a converted surface.

A wide variety of different surfaces can be treated according to any embodiment. One example of a surface is a vessel lumen surface, where the vessel is, for example, a vial, a bottle, a jar, a syringe, a cartridge, a blister package, or an ampoule. For more examples, the surface of the material can be a fluid contact surface of an article of labware, for example a microplate, a centrifuge tube, a pipette tip, a well plate, a microwell plate, an ELISA plate, a microtiter plate, a 96-well plate, a 384-well plate, a centrifuge tube, a chromatography vial, an evacuated blood collection tube, or a specimen tube.

The treated surface of any embodiment can be a coating or layer of PECVD deposited $SiO_xC_yH_z$ or $SiN_xC_yH_z$, in which x is from about 0.5 to about 2.4 as measured by X-ray photoelectron spectroscopy (XPS), y is from about 0.6 to about 3 as measured by XPS, and z is from about 2 to about 9 as measured by Rutherford backscattering spectrometry (RBS). Another example of the surface to be treated is a barrier coating or layer of $SiO_x$, in which x is from about 1.5 to about 2.9 as measured by XPS, alternatively an oxide or nitride of an organometallic precursor that is a compound of a metal element from Group III and/or Group IV of the Periodic Table, e.g. in Group III: Boron, Aluminum, Gallium, Indium, Thallium, Scandium, Yttrium, or Lanthanum, (Aluminum and Boron being preferred), and in Group IV: Silicon, Germanium, Tin, Lead, Titanium, Zirconium, Hafnium, or Thorium (Silicon and Tin being preferred).

The electrical power used to excite the plasma used in plasma treatment in any embodiment, can be, for example, from 1 to 1000 Watts, optionally from 100 to 900 Watts, optionally from 50 to 600 Watts, alternatively 100 to 500 Watts, optionally from 500 to 700 Watts, optionally from 1 to 100 Watts, optionally from 1 to 30 Watts, optionally from 1 to 10 Watts, optionally from 1 to 5 Watts.

The frequency of the electrical power used to excite the plasma used in plasma treatment, in any embodiment, can be any type of energy that will ignite plasma in the plasma zone. For example, it can be direct current (DC) or alternating current (electromagnetic energy) having a frequency from 3 Hz to 300GHz. Electromagnetic energy in this range generally includes radio frequency (RF) energy and microwave energy, more particularly characterized as extremely low frequency (ELF) of 3 to 30 Hz, super low frequency (SLF) of 30 to 300 Hz, voice or ultra-low frequency (VF or ULF) of 300 Hz to 3kHz, very low frequency (VLF) of 3 to 30 kHz, low frequency (LF) of 30 to 300 kHz, medium frequency (MF) of 300 kHz to 3 MHz, high frequency (HF) of 3 to 30 MHz, very high frequency (VHF) of 30 to 300 MHz, ultra-high frequency (UHF) of 300 MHz to 3 GHz, super high frequency (SHF) of 3 to 30 GHz, extremely high frequency (EHF) of 30 to 300 GHz, or any combination of two or more of these frequencies. For example, high frequency energy, commonly 13.56 MHz, is useful RF energy, and ultra-high frequency energy, commonly 2.54 GHz, is useful microwave energy, as two examples of commonly used frequencies.

The plasma exciting energy, in any embodiment, can either be continuous during a treatment step or pulsed multiple times during the treatment step. If pulsed, it can alternately pulse on for times ranging from one millisecond to one second, and then off for times ranging from one millisecond to one second, in a regular or varying sequence during plasma treatment. One complete duty cycle (one "on" period plus one "off" period) can be 1 to 2000 milliseconds (ms), alternatively 1 to 1000 milliseconds (ms), alternatively 2 to 500 ms, alternatively 5 to 100 ms, alternatively 10 to 100 ms long.

Optionally in any embodiment, the relation between the power on and power off portions of the duty cycle can be, for example, power on 1-90 percent of the time, alternatively on 1-80 percent of the time, alternatively on 1-70 percent of the time, alternatively on 1-60 percent of the time, alternatively on 1-50 percent of the time, alternatively on 1-45 percent of the time, alternatively on 1-40 percent of the time, alternatively on 1-35 percent of the time, alternatively on 1-30 percent of the time, alternatively on 1-25 percent of the time, alternatively on 1-20 percent of the time, alternatively on 1-15 percent of the time, alternatively on 1-10 percent of the time, alternatively on 1-5 percent of the time, and power off for the remaining time of each duty cycle.

The plasma pulsing described in Mark J. Kushner, Pulsed Plasma-Pulsed Injection Sources For Remote Plasma Activated Chemical Vapor Deposition, J. APPL. PHYS. 73, 4098 (1993), can optionally be used.

The flow rate of process gas during plasma treatment according to any embodiment can be from 1 to 300 sccm (standard cubic centimeters per minute), alternatively 1 to 200 sccm, alternatively from 1 to 100 sccm, alternatively 1-50 sccm, alternatively 5-50 sccm, alternatively 1-10 sccm.

Optionally in any embodiment, the plasma chamber is reduced to a base pressure from 0.001 milliTorr (mTorr, 0.00013 Pascal) to 100 Torr (13,000 Pascal) before feeding gases. Optionally the feed gas pressure in any embodiment can range from 0.001 to 10,000 mTorr (0.00013 to 1300 Pascal), alternatively from 1 mTorr to 10 Torr (0.13 to 1300 Pascal), alternatively from 0.001 to 5000 mTorr (0.00013 to 670 Pascal), alternatively from 1 to 1000 milliTorr (0.13 to 130 Pascal).

The treatment volume in which the plasma is generated in any embodiment can be, for example, from 100 mL to 50 liters, preferably 8 liters to 20 liters.

The plasma treatment time in any embodiment can be, for example, from 1 to 300 seconds, alternatively 3 to 300 sec., alternatively 30 to 300 sec., alternatively 150 to 250 sec., alternatively 150 to 200 sec., alternatively from 90 to 180 seconds.

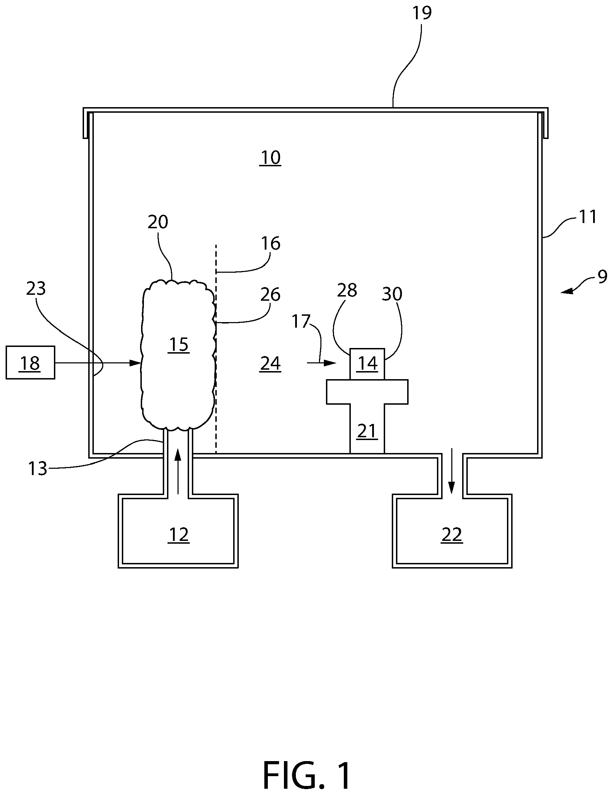

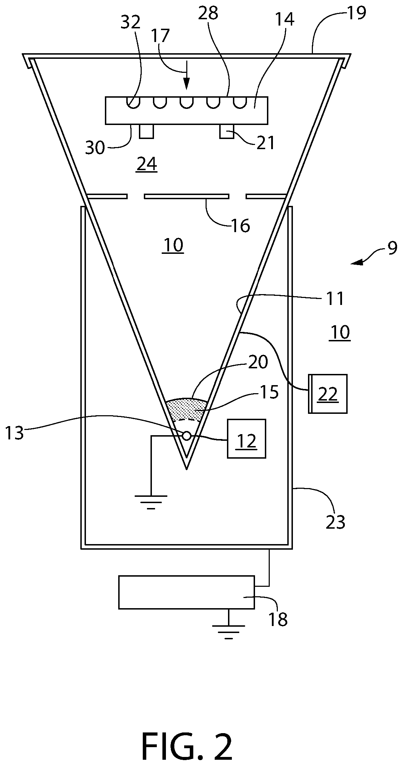

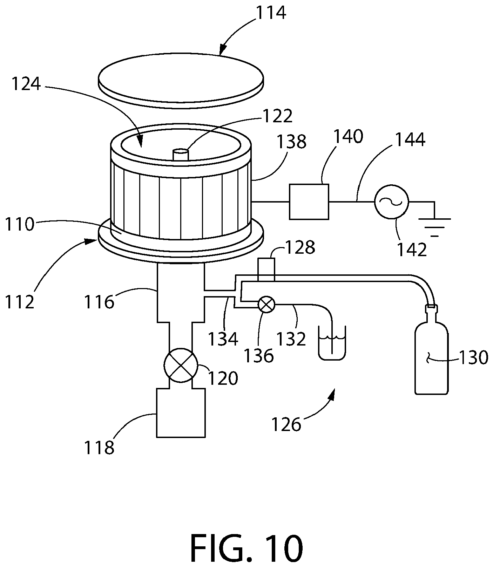

In any embodiment, the plasma treatment apparatus employed can be any suitable apparatus, for example that of Fig. 1, Fig. 7, Fig. 8, or Fig. 10 described in this specification, as several examples. Plasma treatment apparatus of the type that employs the lumen of the vessel to be treated as a vacuum chamber, shown for example in U.S. Patent 7,985,188, Fig. 2, can also be used in any embodiment.

The plasma treatment process of any embodiment optionally can be combined with treatment using an ionized gas. The ionized gas can be, as some examples, any of the gases identified as suitable for plasma treatment. The ionized gas can be delivered in any suitable manner. For example, it can be delivered from an ionizing blow-off gun or other ionized gas source. A convenient gas delivery pressure is from 1-120 psi (pounds per square inch) (6 to 830 kPa, kiloPascals) (gauge or, alternatively, absolute pressure), optionally 50 psi (350 kPa). The water content of the ionized gas can be from 0 to 100%. The treatment with ionized gas can be carried out for any suitable treatment time, for example from 1-300 seconds, optionally for 10 seconds.

After the plasma treatment(s) of any embodiment, the treated surface, for example a vessel lumen surface, can be contacted with an aqueous protein. Some examples of suitable proteins are: mammal serum albumin, for example Bovine Serum Albumin (BSA); Fibrinogen (FBG); Transferrin (TFN), for example blood serotransferrin (or siderophilin, also known as transferrin); lactotransferrin (lactoferrin); milk transferrin; egg white ovotransferrin (conalbumin); and membrane-associated melanotransferrin; Protein A (PrA); Protein G (PrG); Protein A/G; Protein L; Insulin, for example hexameric insulin, monomeric insulin, porcine insulin, human insulin, recombinant insulin and pharmaceutical grades of insulin; Pharmaceutical protein; blood or blood component proteins; or any recombinant form, modification, full length precursor, signal peptide, propeptide, or mature variant of these proteins; or a combination of two or more of these.

Optionally, the converted surface has a protein recovery percentage greater than the protein recovery percentage of the untreated surface for at least one of Bovine Serum Albumin (BSA); Fibrinogen (FBG); Transferrin (TFN), for example blood serotransferrin (or siderophilin, also known as transferrin); lactotransferrin (lactoferrin); milk transferrin; egg white ovotransferrin (conalbumin); and membrane-associated melanotransferrin; Protein A (PrA); Protein G (PrG); Protein A/G; Protein L; Insulin, for example hexameric insulin, monomeric insulin, porcine insulin, human insulin, recombinant insulin and pharmaceutical grades of insulin; pharmaceutical protein; blood or blood component proteins; or any recombinant form, modification, full length precursor, signal peptide, propeptide, or mature variant of these proteins.

A vessel having a plastic substrate treated according to the present invention may be made, for example, from any of the materials defined above. For applications in which clear and glass-like polymers are desired (e.g., for syringes and vials), a cyclic olefin polymer (COP), cyclic olefin copolymer (COC), polymethylmethacrylate, polyethylene terephthalate or polycarbonate may be preferred. Such substrates may be manufactured, e.g., by injection molding or injection stretch blow molding (which is also classified as injection molding in this disclosure), to very tight and precise tolerances (generally much tighter than achievable with glass).

A vessel treated according to the present invention can be a sample tube, e.g. for collecting or storing biological fluids like blood or urine, a syringe (or a part thereof, for example a syringe barrel) for storing or delivering a biologically active compound or composition, e.g., a medicament or pharmaceutical composition, a vial for storing biological materials or biologically active compounds or compositions, a pipe, e.g., a catheter for transporting biological materials or biologically active compounds or compositions, or a cuvette for holding fluids, e.g., for holding biological materials or biologically active compounds or compositions. Other examples of contemplated vessels include well or non-well slides or plates, for example titer plates or microtiter plates (a.k.a. microplates). Other examples of vessels include measuring and delivery devices such as pipettes, pipette tips, Erlenmeyer flasks, beakers, and graduated cylinders. The specific vessels described herein with respect to an actual reduction to practice of the invention are polypropylene 96-well microplates and beakers. However, a skilled artisan would understand that the methods and equipment set-up described herein can be modified and adapted, consistent with the present invention, to accommodate and treat alternative vessels.

The surface of the vessel may be made from the substrate material itself, e.g., any of the thermoplastic resins listed above. Alternatively, the surface may be a pH protective coating or layer of PECVD deposited $SiO_xC_yH_z$ or $SiN_xC_yH_z$, in which x is from about 0.5 to about 2.4 as measured by X-ray photoelectron spectroscopy (XPS), y is from about 0.6 to about 3 as measured by XPS, and z is from about 2 to about 9 as measured by Rutherford backscattering spectrometry (RBS). Another example is the surface is a barrier coating or layer of PECVD deposited $SiO_x$, in which x is from about 1.5 to about 2.9 as measured by XPS, alternatively an oxide or nitride of an organometallic precursor that is a compound of a metal element from Group III and/or Group IV of the Periodic Table, e.g. in Group III: Boron, Aluminum, Gallium, Indium, Thallium, Scandium, Yttrium, or Lanthanum, (Aluminum and Boron being preferred), and in Group IV: Silicon, Germanium, Tin, Lead, Titanium, Zirconium, Hafnium, or Thorium (Silicon and Tin being preferred). Methods and equipment for depositing these coatings or layers are described in detail in WO2013/071138, published May 16, 2013.

Methods according to the present invention employ the use of remote conversion plasma treatment. Unlike direct plasma processing, in the case of remote conversion plasma, neither ions nor electrons of plasma contact the article surface. Neutral species, typically having lower energy, are present in the plasma afterglow, which are sufficiently energetic to react with the article surface, without sputtering or other higher energy chemical reactions induced by ions and electrons. The result of remote conversion plasma is a gentle surface modification without the high energy effects of "direct" plasmas.

Methods according to the present invention employ non-polymerizing gases, such as $O_2$, $N_2$, air, $O_3$, $N_2O$, $H_2$, $H_2O_2$, $NH_3$, Ar, He, Ne, other non-polymerizing gases, and combinations of any of two or more of the foregoing. These may also include non-polymerizing alcohols, non-polymerizing organic acids and non-polymerizing polar organic solvents. Applicant has successfully carried out experiments wherein the first treatment step (non-polymerizing compound step) used Ar, $N_2$, Ar/$O_2$ mix, or $N_2$/$O_2$ mix and a pre-treatment conditioning step with Ar. These and other non-polymerizing gases do not necessarily deposit a coating. Rather, they react with the surface to modify the surface, e.g., to form a converted surface, wherein the converted surface has a biomolecule recovery percentage greater than the biomolecule recovery percentage of the untreated surface. For example, the surface reactions may result in new chemical functional groups on the surface, including carbonyl, carboxyl, hydroxyl, nitrile, amide, amine. It is contemplated that these polar chemical groups increase the surface energy and hydrophilicity of otherwise hydrophobic polymers that an untreated surface may typically comprise. While hydrophobic surfaces are generally good binding surfaces for biomolecules, hydrophilic surfaces, which attract water molecules, facilitate the blocking of biomolecules binding to that surface. While not being bound to this theory of operation, it is contemplated that this mechanism prevents biomolecule binding to surfaces.

Methods according to the present invention are used to reduce the propensity of a substrate surface to cause biomolecules to adhere thereto. Preferably, the methods will reduce biomolecule adhesion across a wide spectrum of biomolecules, including one or more of the following aqueous proteins: mammal serum albumin, for example Bovine Serum Albumin (BSA); Fibrinogen (FBG); Transferrin (TFN), for example blood serotransferrin (or siderophilin, also known as transferrin); lactotransferrin (lactoferrin); milk transferrin; egg white ovotransferrin (conalbumin); and membrane-associated melanotransferrin; Protein A (PrA); Protein G (PrG); Protein A/G; Protein L; Insulin, for example hexameric insulin, monomeric insulin, porcine insulin, human insulin, recombinant insulin and pharmaceutical grades of insulin; Pharmaceutical protein; blood or blood component proteins; or any recombinant form, modification, full length precursor, signal peptide, pro-peptide, or mature variant of these proteins; and a combination of two or more of these.

Fig.1 is a schematic generic view of remote conversion plasma treatment apparatus 9 having common features with each more particular apparatus of Figs. 2, 6, 7, and 8 for carrying out remote conversion plasma treatment according to the invention. Plasma gas from a fluid source 12 capable of supporting the generation of plasma in the plasma zone 15 having a boundary 20 (plasma is defined here as a visible glow discharge) is introduced via a fluid inlet 13 to a plasma zone 15, and plasma energy from a plasma energy source 18 is provided to the plasma zone 15 to generate plasma having a boundary 20 in the plasma zone 15.

The plasma energy used in the method of the present invention broadly can be any type of energy that will ignite plasma in the plasma zone 15. For example, it can be direct current (DC) or alternating current (electromagnetic energy) having a frequency from 3 Hz to 300GHz. Electromagnetic energy in this range generally includes radio frequency (RF) energy and microwave energy, more particularly characterized as extremely low frequency (ELF) of 3 to 30 Hz, super low frequency (SLF) of 30 to 300 Hz, voice or ultra-low frequency (VF or ULF) of 300 Hz to 3kHz, very low frequency (VLF) of 3 to 30 kHz, low frequency (LF) of 30 to 300 kHz, medium frequency (MF) of 300 kHz to 3 MHz, high frequency (HF) of 3 to 30 MHz, very high frequency (VHF) of 30 to 300 MHz, ultra-high frequency (UHF) of 300 MHz to 3 GHz, super high frequency (SHF) of 3 to 30 GHz, extremely high frequency (EHF) of 30 to 300 GHz, or any combination of two or more of these frequencies. For example, high frequency energy, commonly 13.56 MHz, is useful RF energy, and ultra-high frequency energy, commonly 2.54 GHz, is useful microwave energy, as two examples of commonly used frequencies.

The nature of the optional applicator 23 is determined by the frequency and power level of the energy, as is well known. If the plasma is excited by radio waves, for example, the applicator 23 can be an electrode, while if the plasma is excited by microwave energy, for example, the applicator 23 can be a waveguide.

An afterglow region 24 is located outside but near the plasma boundary 20, and contains treatment gas 17. The afterglow region 24 can be the entire treatment volume 10 outside the plasma boundary 20 and within the reaction chamber wall 1 and lid 19, or the afterglow region 24 can be a subset of the treatment volume 10, depending on the dimensions of and conditions maintained in the treatment volume. The treatment gas 17 in the afterglow region 24 is not ionized sufficiently to form plasma, but it is sufficiently energetic to be capable of modifying a surface that it contacts, more so than the same gas composition at the same temperature and pressure in the absence of the plasma.

It will be understood by a skilled person that some gas compositions are sufficiently chemically reactive that they will modify a substrate in the apparatus 9 when plasma is absent. The test for whether a region of, or adjacent to, remote conversion plasma treatment apparatus is within the afterglow, for given equipment, plasma, gas feed, and pressure or vacuum conditions producing a visible glow discharge outside the region, is whether a substrate located in the region under the given equipment, plasma, gas feed, and pressure is modified compared to a substrate exposed to the same equipment, gas feed and pressure or vacuum conditions, when no plasma is present in the plasma zone as the result of the absence of or insufficiency of the plasma energy 18.

Remote conversion plasma treatment is carried out by providing plasma in the plasma zone 15, which generates an afterglow in the afterglow region or remote conversion plasma (two terms for the same region) 24, which contacts and modifies a substrate placed at least partially in the afterglow region 24.

As one option of performing the method of the present invention in the remote conversion plasma treatment apparatus, the plasma gas enters the plasma zone, is excited to form plasma, then continues downstream to the afterglow region 24 where it has less energy, is then defined as treatment gas 17, and contacts the substrate. In other words, at least a portion of the gas flows through the plasma zone 15, is energized to form plasma, and continues to the afterglow region 24, becoming more energetic in the plasma zone 15 and less energetic by the time it enters the afterglow region 24 (but still energized compared to the gas before entering the plasma zone 15). Where this option is adopted, the plasma and the afterglow region 24 are in gas communication and at least some of the same gas is fed through both zones. Optionally, as where plasma is not generated in the entire cross-section of flowing gas, some of the gas may bypass the plasma by staying outside the boundary 20 of the plasma zone 15 and still flow through the afterglow region 24, while other gas flows through both the plasma zone 15 and the afterglow region 24.

As another option in the remote conversion plasma treatment apparatus, the plasma gas can be different molecules from the treatment gas 17 (though the plasma gas and treatment gas may either have identical compositions or different compositions), and the plasma gas remains in or is fed through only the plasma zone 15 and not the afterglow region 24, while the treatment gas is energized by the plasma gas but is separate from the plasma gas and while in the afterglow region 24 is not energized sufficiently to form plasma.

The nature of the applicator 23 can vary depending on the application conditions, for example the power level and frequency of the plasma energy 18. For example, the applicator can be configured as an electrode, antenna, or waveguide.

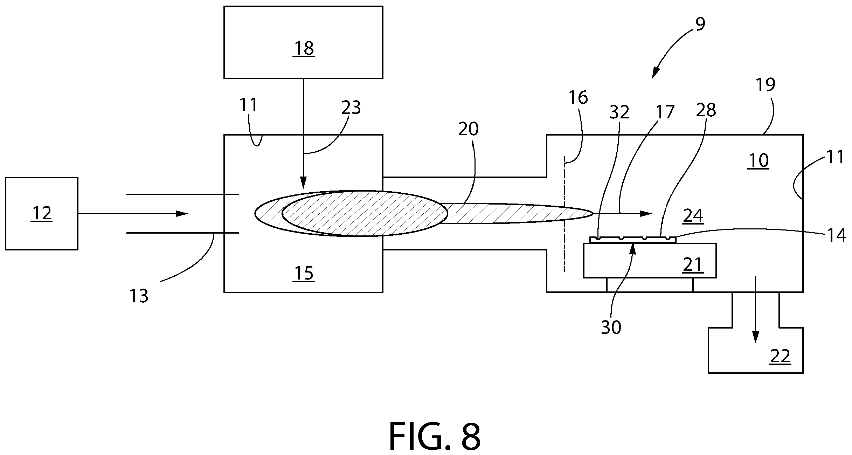

Optionally, a shield 16 may be placed between the plasma and at least a portion of the substrate 14 in the treatment area to prevent the plasma from contacting or coming undesirably close to the substrate 14 or unevenly affecting the substrate 14. For one example, the optional shield 16 in Fig. 1 can be perforated to allow gas flow through it, particularly flow of the neutral species forming the afterglow, but the shield 16 is configured or equipped, suitably for the choice of plasma-forming energy, to prevent the plasma from penetrating the shield. For example, the perforations may be sized or the shield can be electrically biased such that the plasma-forming energy or the plasma cannot pass through it. This arrangement has the advantage that, if the plasma zone has a substantial area intersecting with the shield, the substantial area optionally is flattened so the plasma boundary 20 has a "flat spot" 26, illustrated in Fig. 1, which can be placed parallel to the surface of the substrate to be treated so they are equidistant over a substantial area, instead of the plasma terminating in a tapered tail that extends much closer to one portion of the substrate 14 than to other parts of the substrate 14 not aligned with the tail, illustrated in Fig. 8.

Another shield option is that the shield can be made such that it passes neither gas nor plasma, serving as an obstruction of the direct path between some or all of the plasma and some or all of the treatment area. The obstruction can fill less than all of the gas cross-section flowing from the plasma zone 15 to the afterglow region 24, so non-ionized gas can flow around the shield and reach the afterglow region 24 by a circuitous path, while plasma cannot either circumvent or pass through it.

Yet another shield option is that the substrate 14 to be treated can be positioned in the apparatus during treatment such that one portion of a substrate 14 that can withstand contact with plasma is exposed to the plasma, shielding from the plasma another portion of the substrate 14 or another substrate receiving remote conversion plasma treatment.

Still another shield option is that the gas flow path through the plasma and treatment area can be sharply bent, for example turning a 90 degree corner between the plasma and treatment area, so the wall of the apparatus itself shields the treatment area from line-of-sight relation to the plasma under certain treatment conditions.

The substrate orientation in the treatment volume can vary, and the substrate, applicator, gas and vacuum sources can optionally be arranged to provide either substantially even or uneven exposure to remote conversion plasma across a substrate.

Another option is that the substrate itself can serve as the reactor wall or a portion of the reactor wall, so treatment gas 17 introduced into reactor treats the portion of the substrate serving as the reactor wall.

Another option is the introduction of a second non-polymerizing gas, functioning as diluent gas, into the reactor, in addition to the non-polymerizing compound or water vapor which is the active agent of the treatment gas 17. Diluent gases are defined as gases introduced at the fluid inlet 13 that do not materially interact with the substrate 14 to the extent they find their way into the treatment gas 17, given the treatment apparatus and conditions applied. Diluent gases can either participate or not participate in formation of the plasma. The diluent gas can be introduced through the inlet 13 or elsewhere in the reactor. Diluent gases can be added at a rate from 1% to 10,000% by volume, optionally 10% to 1000% by volume, optionally 100% to 1000% by volume, of the rate of addition of the non-polymerizing compound or water vapor.

As another option, some or all of the non-polymerizing compound or water vapor can be added to the treatment volume 10 in such a manner as to bypass the plasma zone 15 en route to the treatment gas 17.

Fig. 2 shows another embodiment of the apparatus of Fig. 1. The apparatus again can be used for carrying out the remote conversion plasma treatment according to the present invention. The chamber of this embodiment comprises a treatment volume 10 defined and enclosed by a reaction chamber wall 11, which optionally is not electrically conductive. The treatment volume 10 is supplied with a fluid source 12 (in this instance, a tubular fluid inlet 13 projecting axially into the treatment volume 10, however other fluid sources are contemplated, e.g., "shower head" type fluid sources). Optionally, the treatment volume 10 can be defined by a treatment chamber wall 11 or by the lumen within a vessel or other article to be treated. Feed gases are fed into the treatment volume 10. The plasma reaction chamber comprises as an optional feature a vacuum source 22 for at least partially evacuating the treatment volume 10 compared to ambient pressure, for use when plasma treating at reduced pressure, although plasma treating under suitable conditions at ambient atmospheric pressure or at a pressure higher than ambient atmospheric pressure is also contemplated.

The plasma reaction chamber also comprises an optional outer applicator 23, here in the form of an electrode surrounding at least a portion of the plasma reaction chamber. A radio frequency (RF) plasma energy source 18 is coupled to the reaction chamber by an applicator 23 and provides power that excites the gases to form plasma. The plasma forms a visible glow discharge 20 that optionally is limited to a close proximity to the fluid source 12.

Microplates 14 optionally can be oriented such that the surfaces of the microplates 14 on which treatment is desired (the surface that is configured and intended to contact/hold a biomolecule-containing solution) face the fluid source 12. However, the surfaces to be treated can also or instead face away from the fluid source 12, as shown in Fig. 2. In addition, in the illustrated embodiment the microplate 14 is shielded with a shield 16 to block the microplate 14 from being in the direct "line of sight" of (i.e. having an unobstructed path to) the fluid source 12. As an example, the respective surfaces of the microplates 14 can be positioned a horizontal distance of approximately 2.75 inches (7 cm) from the fluid source, although operation is contemplated with placement of the microplate 14 surfaces at a horizontal distance of from ½ to 10 inches (1 to 25 cm), optionally 2.5 to 5.5 inches (6 to 14 cm) from the fluid source. In this manner, the process relies on remote conversion plasma (as opposed to direct plasma) to treat the microplates' 14 surfaces. In this example, the system has a capacity of 20 parts (microplates) per batch at a total process time of eight minutes per batch.

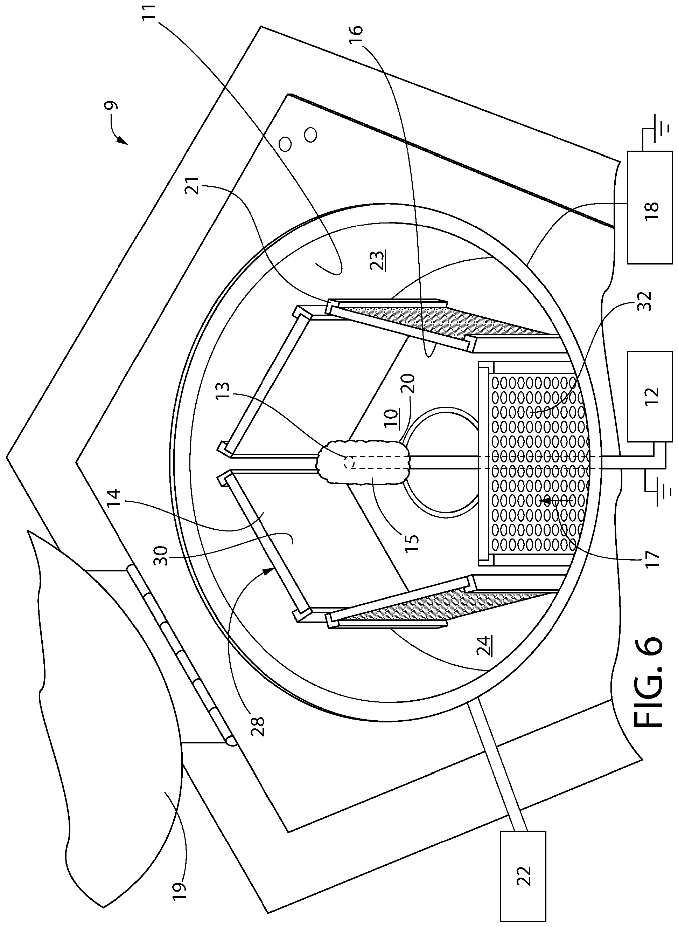

Fig. 6 shows another embodiment of the apparatus of Fig. 1. The process used to treat the microplates in FIG. 6 uses a radio-frequency (RF) plasma system. The system has a gas delivery input, a vacuum pump and RF power supply with matching network. The microplates are shown oriented with the front surfaces containing the wells 32 facing away from and shielded from the plasma along the perimeter of the chamber.

These details are illustrated in Fig. 6, where there is shown another exemplary setup having all the elements of the apparatus of Fig. 2 for use in a plasma reaction chamber for carrying out remote conversion plasma treatment according to the present invention. The chamber comprises a treatment volume 10 defined and enclosed by a reaction chamber wall 11 having a fluid source 12 (in this instance, a tubular fluid inlet 13 projecting axially into the treatment volume 10, however other fluid sources are contemplated, e.g., "shower head" type fluid sources). The reaction chamber wall 11 in this embodiment is provided with a removable lid 13 that is openable to allow substrates to be inserted or removed and sealable to contain the process and, optionally, evacuate the treatment volume. The fluid source 12 is made of metallic material, electrically grounded, and also functions as an applicator, in the form of an inner electrode. As is well known, the plasma alternatively can be generated without an inner electrode.

Feed gases are fed into the treatment volume 10. The plasma reaction chamber comprises an optional feature of a vacuum source 22 for at least partially evacuating the treatment volume 10. The plasma reaction chamber wall 11 also functions as an applicator 23 in the form of an outer applicator or electrode surrounding at least a portion of the plasma reaction chamber. A plasma energy source 18, in this instance a radio frequency (RF) source, is coupled to applicators 23 defined by the reaction chamber wall 24 and the fluid source 12 to provide power that excites the gases to form plasma. The plasma zone 15 forms a visible glow discharge that is limited by the plasma boundary 20 in close proximity to the fluid source 12. The afterglow region also known as a remote conversion plasma region 24 is the region radially or axially outside the boundary 20 of the visible glow discharge and extending beyond the substrates treated.

Microplates 14 having front surfaces 28 and back surfaces 30 are oriented such that the wells 32 on the front surfaces of the microplates 14 on which treatment is desired (the front surface that is configured and intended to contact/hold a biomolecule-containing solution) face away from the fluid source 12 and the back surfaces 30 face toward the fluid source 12. The front surfaces 28 of the microplates 14 are shielded by their own back surfaces 30 to block the microplate front surfaces 28 from being in the direct "line of sight" of the fluid source 12. In this manner, the process relies on remote conversion plasma (as opposed to direct plasma) to treat the surfaces of the wells 32.

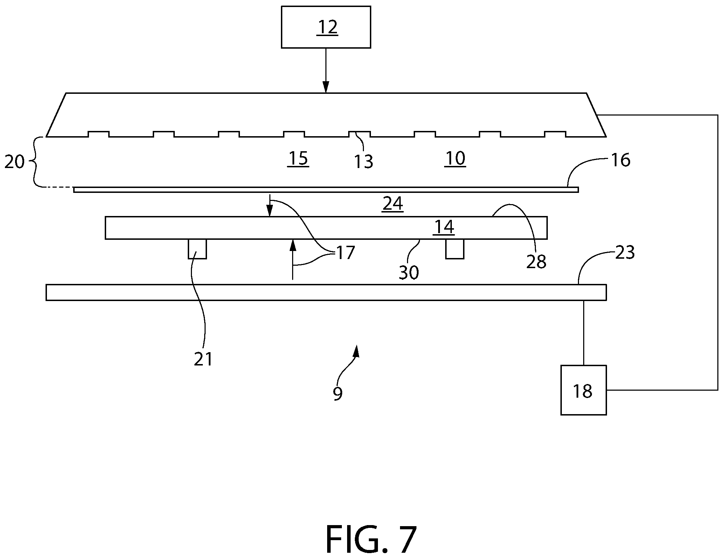

Fig. 7 shows another embodiment of the apparatus of Fig. 1, having corresponding features. The embodiment of Fig. 7 provides a "shower head" fluid inlet 13 and a plate electrode as the applicator 23 that provide more uniform generation and application of treatment gas 17 over a wider area of the substrate 14.

Fig. 8 shows another embodiment of the apparatus of Fig. 1, having corresponding features. The embodiment of Fig. 8 provides microwave plasma energy 18 delivered through an applicator 23 configured as a waveguide. In this embodiment the plasma zone 15 and substrate support 21 are provided in separate vessels connected by a conduit.

Optionally, the converted surface has a biomolecule recovery percentage greater than the biomolecule recovery percentage of the untreated surface for at least one of Bovine Serum Albumin (BSA); Fibrinogen (FBG); Transferrin (TFN), for example blood serotransferrin (or siderophilin, also known as transferrin); lactotransferrin (lactoferrin); milk transferrin; egg white ovotransferrin (conalbumin); and membrane-associated melanotransferrin; Protein A (PrA); Protein G (PrG); Protein A/G; Protein L; Insulin, for example hexameric insulin, monomeric insulin, porcine insulin, human insulin, recombinant insulin and pharmaceutical grades of insulin; pharmaceutical protein; blood or blood component proteins; or any recombinant form, modification, full length precursor, signal peptide, propeptide, mature variant of these proteins and a combination of two or more of these.

The plasma treatment process according to the present invention comprises, consists essentially of, or consists of the two treatment steps using remote conversion plasma defined in the claims. It should be understood that additional steps prior to, between or after the aforementioned steps may be added.

Optional process parameter ranges for the first treatment step (non-polymerizing compound plasma step) and second treatment step (water vapor plasma step) of the first more detailed embodiment are set forth in Table 1.

Table 1 - Process parameter ranges for plasma treatment

| Non-Polymerizing Compound Plasma Step | | Water Vapor Plasma Step | |
|---|---|---|---|
| Power (W) | 50-600 | Power (W) | 50-600 |
| Gas Flow rate (sccm) | 5-50 | $H_2O$ Flow rate (sccm) | 1-10 |
| Time (minutes) | 0.5-5 | Time (minutes) | 0.05-5 |
| Pressure (mTorr) | 0-1,000 | Pressure (mTorr) | 0-5,000 |

Optionally, no pretreatment step is required prior to the non-polymerizing gas plasma step.

Optionally, the remote conversion plasma used to treat a substrate surface may be RF generated plasma.

Optionally, the treatment volume in a plasma reaction chamber may be from 100 mL to 50 liters, preferably 8 liters to 20 liters for certain applications. Optionally, the treatment volume may be generally cylindrical, although other shapes and configurations are also contemplated.

Various aspects will be illustrated in more detail with reference to the following Examples.

**Testing of all embodiments**

The following protocol was used to test the plates in all embodiments, except as otherwise indicated in the examples:

Purpose: The purpose of this experiment was to determine the amount of protein binding over time to a surface coated microtiter plate.

Materials: BIOTEK® Synergy H1 Microplate Reader and BIOTEK Gen5® Software, MILLIPORE® MILLIQ® Water System (sold by Merck KGAA, Darmstadt, Germany), MILLIPORE® Direct Detect Spectrometer, ALEXA FLUOR® 488 Labeled Proteins (Bovine Serum Albumin (BSA), Fibrinogen (FBG), Transferrin (TFN), Protein A (PrA) and Protein G (PrG), sold by Molecular Probes, Inc., Eugene, Oregon USA), 10X Phosphate Buffered Saline (PBS), NUNC® Black 96-well Optical Bottom Plates, 1 L Plastic Bottle, 25-100 mL Glass Beakers, Aluminum Foil, 1-10 mL Pipette, 100-1000 µL Pipette, 0.1-5 µL Pipette, 50-300 µL Multichannel Pipette.

The selected proteins, one or more of those listed above, were tested on a single surface coated microplate. Each protein was received as a fluorescently labeled powder, labeled with ALEXA FLUOR® 488:

- 5 mg of BSA: 66,000 Da

- 5 mg of FBG: 340,000 Da

- 5 mg of TFN: 80,000 Da

- 1 mg of PrA: 45,000 Da

- 1 mg of PrG: 20,000 Da

Once received, each vial of protein was wrapped in aluminum foil for extra protection from light and labeled accordingly, then placed into the freezer for storage.

A solution of 1 × PBS (phosphate buffer solution) was made from a stock solution of 10× PBS: 100mL of 10× PBS was added to a plastic 1L bottle, followed by 900mL of distilled water from the MILLIPORE® Q-pod, forming 1× PBS. Using a 100-1000 μL pipette, 1000 μL of 1× PBS was pipetted into each vial of protein separately, to create protein solutions. Each vial was then inverted and vortexed to thoroughly mix the solution.

Each protein was then tested on the MILLIPORE® Direct Detect to get an accurate protein concentration. Using a 0.1-5 μL pipette, a 2 μL sample of PBS was placed on the first spot of the Direct Detect reading card and marked as a blank in the software. A 2 μL sample of the first protein was then placed onto the remaining 3 spots and marked as samples. After the card was read, an average of the 3 protein concentrations was recorded in mg/mL. This was repeated for the remaining 4 proteins. The protein solutions were then placed into the refrigerator for storage.

A standard curve was prepared with 1X PBS for each protein. The standard curve started at 25 nM and a serial 2× dilution was performed to obtain the other tested concentrations, for example one or more of 12.5 nM, 6.25 nM, 3.125 nM and 1.5625 nM. The 12.5 nM solution prepared from the standard curve was used for testing.

Once the dilutions for all tested proteins were done, the standard curve for each protein was prepared and tested as follows. 25 100-mL glass beakers were set into rows of 5. Each beaker was wrapped in aluminum foil and labeled with the name of the protein the curve corresponded to and the concentration of the solution in the beaker. Row 1 was the standard curve for BSA; row 2, FBG; row 3, TFN; row 4, PrA; row 5, PrG. Therefore the first row was labeled as follows: BSA 25 nM, BSA 12.5 nM, BSA 6.25 nM, BSA 3.125 nM, BSA 1.56 nM.

After a standard curve was made, it was tested using the microplate reader, then the next standard curve was made and tested, and so on.

The BIOTEK® Synergy H1 microplate reader and BIOTEK Gen5® software were used for analysis.

After the first standard curve was prepared, it was ready to be tested on the Synergy H1. Using a 50-300 μL multichannel pipette, 200 μL of 1× PBS was pipetted into wells A1-A4 of a black optical bottom microplate. Then, 200 μL of the 25 nM solution was pipetted into wells B1-B4, 200 μL of 12.5 nM solution was pipetted into wells C1-C4, 200 μL of 12.5 nM solution was pipetted into wells D1-D4, 200 μL of 12.5 nM solution was pipetted into wells E1-E4, 200 μL of 12.5 nM solution was pipetted into wells F1-F4, and 200 μL of 12.5 nM solution was pipetted into wells G1-G4. A similar procedure was used to fill the wells with other dilutions of the protein solution.

Once the microplate was filled with solution, it was wrapped in aluminum foil and the sections and time points were labeled.

After 1.5 hours, using a 50-300 μL multichannel pipette and poking through the aluminum foil, 200 μL of BSA solution was pipetted from the wells in the 1.5 hr column (column 1) and placed into a black optical bottom microplate. The black microplate was placed into the microplate tray. The other four proteins were then read the same way by opening their corresponding experiments. The same thing was done after 2.5 hours, 4.5 hours and 24 hours. After the 24hr read, "Plate → Export" was then selected from the menu bar. An excel spreadsheet appeared and could then be saved in the desired location with the desired name.

Using the data produced by the BIOTEK Gen5® software, the 12.5 nM solution concentrations from both the standard curve and SPL1 were averaged. The concentrations in the 4 wells at 1.5 hr were averaged. This was then done for 2.5 hr, 4.5 hr and 24 hr also. The average concentration at each time point was then divided by the average concentration of the 12.5 nM solution from the beginning and multiplied by 100 to get a percent recovery at each time point:

$$\% \text{ Recovery @ } 1.5hr = [\text{AVG. BSA } 1.5 \text{ hr}] / [\text{AVG } 12.5 \text{ nM solution}] * 100$$

EXAMPLES

**Example 1**

Polypropylene 96-well microplates were plasma treated. The process used to treat the microplates used a radio-frequency (RF) plasma system. The system had a gas delivery input, a vacuum pump and RF power supply with matching network. The microplates were oriented facing away from and shielded from the plasma along the perimeter of the chamber. These details are illustrated in Fig. 2. The shielding resulted in remote plasma treatment in which the ratio between the radiant density at the remote points on the surfaces of the microplates and the brightest point of the plasma discharge was less than 0.25.

The two step remote conversion plasma process used according to this example is summarized in Table 2:

Table 2 - Process parameters for plasma treatment per Example 1

| Process Step 1-Conditioning Plasma | | Process Step 2- Conversion Plasma | |
| --- | --- | --- | --- |
| Power (W) | 500 | Power (W) | 500 |
| $O_2$ Flow rate (sccm) | 50 | $H_2O$ Flow rate (sccm) | 5 |
| Time (minutes) | 5 | Time (minutes) | 3 |
| Pressure (mTorr) | 50 | Pressure (mTorr) | 80-120 |

The biomolecule binding resistance resulting from this remote conversion plasma process on the surface of the treated microplates was analyzed by carrying out the Testing of All Embodiments. The percent recovery is the percentage of the original concentration of the protein remaining in solution, i.e., which did not bind to the surface of a microplate.

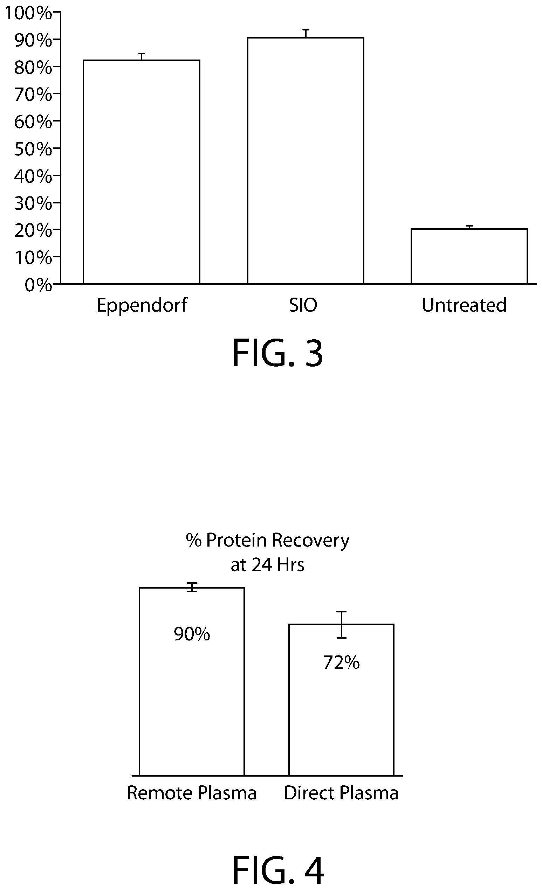

In this testing, samples of three different types of microplates were tested for percent recovery. The samples included: (1) untreated polypropylene microplates ("Untreated" samples); (2) polypropylene microplates treated as described in Example 1 ("SIO" samples); and (3) EPPENDORF brand microplates ("EPPENDORF" samples). The bar chart in Fig. 3 shows the results of this comparative testing. As Fig. 3 illustrates, the SIO samples had a 60% increase in biomolecule recovery compared to the Untreated Samples and an 8-10% increase in biomolecule recovery compared to the EPPENDORF samples.

Accordingly, remote conversion plasma treatment according to the present invention has been demonstrated to result in lower biomolecule adhesion (or the inverse, higher biomolecule recovery) than other known methods. In fact, the comparative data of the SIO samples and the EPPENDORF samples were particularly surprising, since EPPENDORF has been considered the industry standard in protein resistant labware. The SIO samples' 8-10% increase in efficacy compared to the EPPENDORF samples represents a marked improvement compared to the state of the art.

## Example 2

In this example, the SIO samples of Example 1 were compared to the same microplates that were treated with same process steps and conditions as the SIO, except (and this is an important exception), the second samples were treated with direct plasma instead of remote conversion plasma (the "Direct Plasma" samples). Surprisingly, as shown in Fig. 4, the Direct Plasma samples had a biomolecule recovery percentage after 24 hours of 72%, while the SIO samples (which were treated under the same conditions/process steps except by remote conversion plasma) had a biomolecule recovery percentage after 24 hours of 90%. This remarkable step change demonstrates the unexpected improvement resulting solely from use of remote conversion plasma in place of direct plasma.

## Example 3

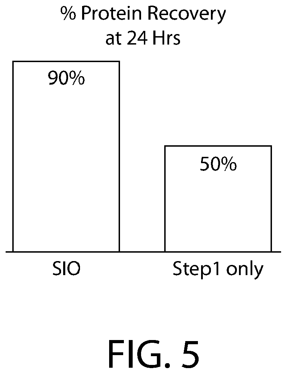

In this example, the SIO samples of Example 1 were compared to the same microplates that were treated with only the first treatment step of the method of the present invention (i.e., the non-polymerizing compound plasma step or conditioning plasma treatment) without the second treatment step (water vapor plasma step or conversion plasma treatment) ("Step 1 Only" samples). As shown in Fig. 5, Step 1 Only samples had a biomolecule recovery percentage after 24 hours at about 25 °C (the aging of all protein samples in this specification is at 25°C unless otherwise indicated) of 50%, while the SIO samples (which were treated under the same conditions/process steps except also by remote conversion plasma) had a biomolecule recovery percentage after 24 hours of 90%. Accordingly, using both steps of the method according to the present invention results in significantly improved biomolecule recovery percentage than using only the first treatment step alone.

## Example 4 (prophetic)

A further contemplated optional advantage of the present invention is that it provides high levels of resistance to biomolecule adhesion without a countervailing high extractables profile. For example, a leading brand of labware made by EPPENDORF is resistant to biomolecule adhesion by virtue of a chemical additive, which has a propensity to extract from the substrate and into a solution in contact with the substrate. By contrast, the method of the present invention does not rely on chemical additives mixed into a polymer substrate to give the substrate its biomolecule adhesion resistant properties. Moreover, processes according to the present invention do not result in or otherwise cause compounds or particles to extract from a treated substrate. Applicant has further determined that the pH protective chemistries described herein, when deposited on a substrate (providing a surface for treatment), do not result in or otherwise cause compounds or particles to extract from a treated surface.

Accordingly, in one optional aspect, the present invention is directed to a method for treating a surface of a plastic substrate, also referred to as a material, to form a converted surface wherein the converted surface has a biomolecule recovery percentage greater than the biomolecule recovery percentage of the surface prior to treatment according to the method, and wherein the method does not materially increase the extractables profile of the substrate. Applicants contemplate that this would bear out in actual comparative tests.

**Example 5**

A test similar to Example 1 was carried out to compare the biomolecule recovery from untreated polypropylene (UTPP) laboratory beakers, remote conversion plasma treated polypropylene (TPP) laboratory beakers, and untreated glass laboratory beakers. The biomolecules used were 12 nM dispersions of lyophilized BSA, FBG, TFN, PrA, and PrG.

In a first trial, the biomolecule dispersion was made up in the beaker and aspirated several times to mix it. The biomolecule recovery was measured in relative fluorescence units (RFU). The initial RFU reading (0 min) was taken to establish a 100% recovery baseline, then the biomolecule dispersion in the beaker was stirred for Imin with a pipet tip, after which it was allowed to remain on the laboratory bench undisturbed for the remainder of the test. The biomolecule recovery was measured initially, then a sample was drawn and measured for percentage biomolecule recovery at each 5-minute interval. The results are shown in Table 3.

**Table 3 - Polypropylene beaker trial**

| UTPP BSA | | UTPP FBG | | UTPP TFN | | UTPP PrA | | UTPP PrG | |
|---|---|---|---|---|---|---|---|---|---|
| Time (min) | %R | Time (min) | %R | Time (min) | %R | Time (min) | %R | Time (min) | %R |
| 0 | 100% | 0 | 100% | 0 | 100% | 0 | 100% | 0 | 100% |
| 1 | 99% | 1 | 99% | 1 | 98% | 1 | 100% | 1 | 101% |
| 5 | 98% | 5 | 97% | 5 | 94% | 5 | 98% | 5 | 99% |
| 10 | 99% | 10 | 99% | 10 | 93% | 10 | 99% | 10 | 100% |
| 15 | 98% | 15 | 92% | 15 | 90% | 15 | 97% | 15 | 99% |
| 20 | 99% | 20 | 98% | 20 | 88% | 20 | 98% | 20 | 101% |
| 25 | 99% | 25 | 97% | 25 | 85% | 25 | 96% | 25 | 98% |
| 30 | 98% | 30 | 96% | 30 | 85% | 30 | 96% | 30 | 99% |

| TPP BSA | | TPP FBG | | TPP TFN | | TPP PrA | | TPP PrG | |
|---|---|---|---|---|---|---|---|---|---|
| Time (min) | %R | Time (min) | %R | Time (min) | %R | Time (min) | %R | Time (min) | %R |
| 0 | 100% | 0 | 100% | 0 | 100% | 0 | 100% | 0 | 100% |
| 1 | 104% | 1 | 101% | 1 | 100% | 1 | 104% | 1 | 104% |
| 5 | 104% | 5 | 100% | 5 | 96% | 5 | 104% | 5 | 104% |
| 10 | 106% | 10 | 101% | 10 | 96% | 10 | 104% | 10 | 104% |
| 15 | 104% | 15 | 99% | 15 | 94% | 15 | 102% | 15 | 104% |
| 20 | 106% | 20 | 100% | 20 | 94% | 20 | 104% | 20 | 104% |
| 25 | 103% | 25 | 98% | 25 | 91% | 25 | 102% | 25 | 101% |
| 30 | 105% | 30 | 98% | 30 | 90% | 30 | 103% | 30 | 103% |

A second trial, with results shown in Table 4, was carried out in the same manner as the first trial except that glass beakers, not treated according to the present invention, were used as the substrate.

**Table 4 - Glass beaker trial**

| Glass BSA | | Glass FBG | | Glass TFN | | Glass PrA | | Glass PrG | |
|---|---|---|---|---|---|---|---|---|---|
| Time (min) | %R | Time (min) | %R | Time (min) | %R | Time (min) | %R | Time (min) | %R |
| 0 | 100% | 0 | 100% | 0 | 100% | 0 | 100% | 0 | 100% |
| 1 | 100% | 1 | 100% | 1 | 100% | 1 | 105% | 1 | 99% |
| 4 | 99% | 4 | 88% | 4 | 98% | 4 | 103% | 4 | 97% |
| 7 | 100% | 7 | 99% | 7 | 97% | 7 | 104% | 7 | 98% |
| 8 | 98% | 8 | 98% | 8 | 96% | 8 | 102% | 8 | 96% |
| 10 | 98% | 10 | 98% | 10 | 95% | 10 | 100% | 10 | 97% |
| 15 | 96% | 15 | 95% | 15 | 92% | 15 | 100% | 15 | 94% |
| 20 | 97% | 20 | 96% | 20 | 92% | 20 | 101% | 20 | 93% |
| 25 | 96% | 25 | 93% | 25 | 88% | 25 | 95% | 25 | 91% |
| 30 | 94% | 30 | 93% | 30 | 87% | 30 | 98% | 30 | 92% |

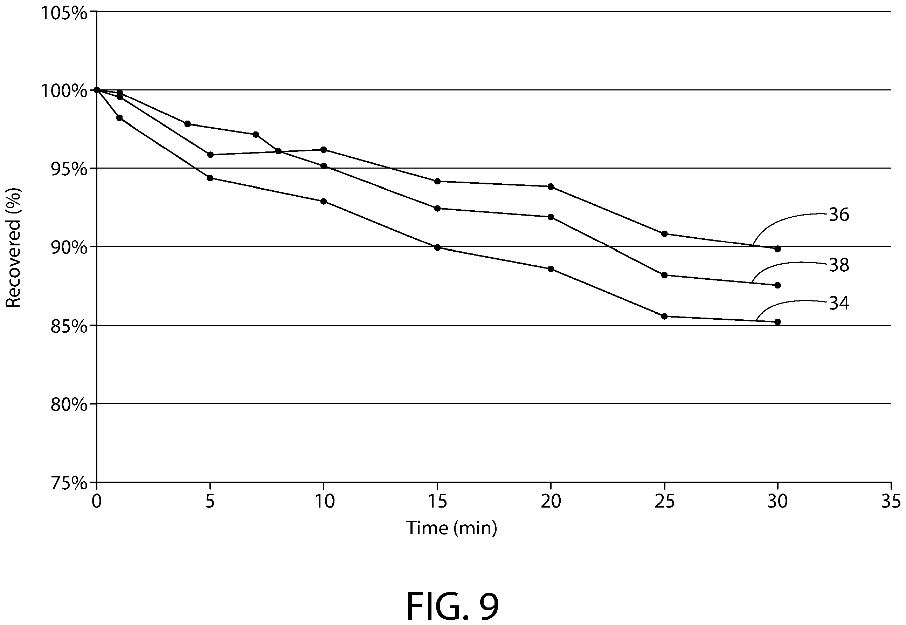

Fig. 9 plots the TFN results in the Tables above, showing plots 34 for the untreated polypropylene beaker, 36 for the treated polypropylene beaker, and 38 for glass. As Fig. 9 shows, the treated polypropylene beaker provided the highest biomolecule recovery after 10 to 30 minutes, glass produced a lower biomolecule recovery after 10 to 30 minutes, and the untreated polypropylene beaker provided the lowest biomolecule recovery at all times after the initial measurement.

**Example 6**

A test similar to Example 1 was carried out to compare the biomolecule recovery from multiwell polypropylene plates of two types, versus protein concentration, after 24 hours of contact between the protein and the plate. "SIO" plates were molded from polypropylene and plasma treated according to Example 1. "CA" (Competitor A) plates were commercial competitive polypropylene plates provided with a coating to provide reduced non-specific protein binding.

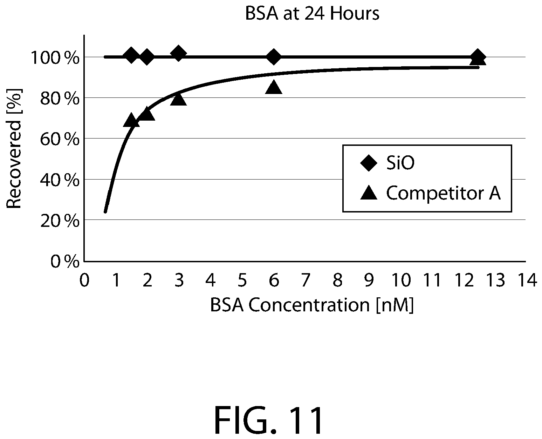

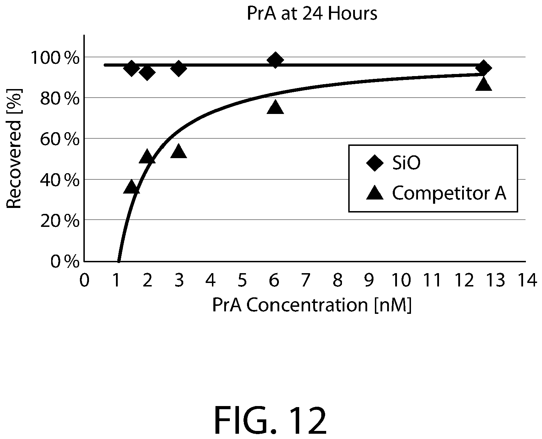

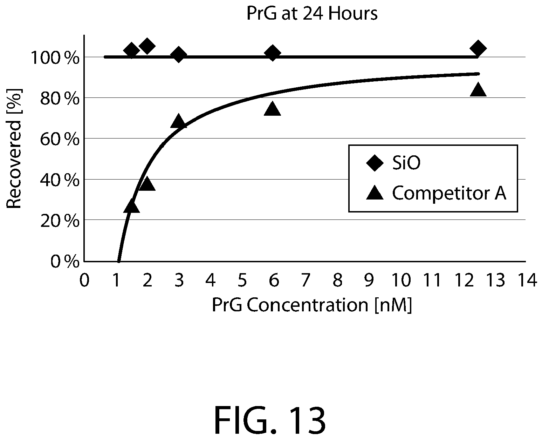

The results are provided in Table 5 and FIGS. 11-13 showing that essentially all the protein of each type was recovered from the SIO plates at all tested concentrations, so the recovery was independent of concentration. In contrast, the protein recovery from the CA plates depended strongly on the concentration, particularly at lower concentrations

**Table 5**

*RECOVERY@24 hrs in* % (96-Well 1000 μL Deep Well Plate)

| Concentration (nM) | Plate | BSA | PrA | PrG |
|---|---|---|---|---|
| 1.5 | SIO | 101 | 94 | 103 |
| 2 | SIO | 100 | 92 | 105 |
| 3 | SIO | 102 | 94 | 101 |
| 6 | SIO | 100 | 98 | 102 |
| 12.5 | SIO | 100 | 94 | 104 |
| 1.5 | CA | 70 | 37 | 27 |
| 2 | CA | 73 | 52 | 38 |
| 3 | CA | 80 | 54 | 69 |
| 6 | CA | 86 | 76 | 75 |
| 12.5 | CA | 100 | 87 | 84 |

**Example 7**

A test similar to Example 1 was carried out to compare the biomolecule recovery from "SIO" plates and "CA" plates of the types described in Example 6. The biomolecules used were 1.5 or 3 nM dispersions of lyophilized BSA, FBG, TFN, PrA, and PrG.

The conditions and results are shown in Table 6. For the BSA, PrA, PrG, and TFN proteins, the SIO plates provided substantially superior protein recovery, compared to the CA plates. For the FBG protein, the SIO plates provided better protein recovery than the CA plates.

**Table 6**
**RECOVERY@72 hrs in** % (96 Well 350 μL (SIO) or 500 μL (CA) Shallow Plate)

| Concentration (nM) | Plate | BSA | FBG | TFN | PrA | PrG |
|---|---|---|---|---|---|---|
| 1.5 | SIO | 104 | 85 | 79 | 99 | 101 |
| 3 | SIO | 100 | 85 | 71 | 93 | 98 |
| 1.5 | CA | 69 | 77 | 45 | 44 | 39 |
| 3 | CA | 74 | 83 | 38 | 60 | 66 |

**Example 8**

A test similar to Example 7 was carried out to compare 96-well, 500 μL SIO and CA plates. The conditions and results are shown in Table 7. For the BSA, PrA, PrG, and TFN proteins, as well as the 1.5 nM concentration of FBG, the SIO plates provided substantially superior protein recovery, compared to the CA plates. The 3 nM concentration of FBG was anomalous.

**Table 7**
**RECOVERY@72 hrs in** % (96 Well 500 μL Deep Well Plate)

| Concentration (nM) | Plate | BSA | FBG | TFN | PrA | PrG |
|---|---|---|---|---|---|---|
| 1.5 | SIO | 101 | 85 | 68 | 93 | 104 |
| 3 | SIO | 96 | 74 | 71 | 91 | 104 |
| 1.5 | CA | 69 | 77 | 45 | 44 | 39 |
| 3 | CA | 74 | 83 | 38 | 60 | 66 |

**Example 9**

A test similar to Example 7 was carried out to compare 96-well, 1000 μL SIO and CA plates. The conditions and results are shown in Table 8. For the BSA, PrA, and PrG proteins, the SIO plates provided substantially superior protein recovery, compared to the CA plates. The FBG proteins did not demonstrate substantially superior protein recovery.

**Table 8**
**RECOVERY@72 hrs in** % (96 Well 1000 μL Deep Well Plate)

| Concentration (nM) | Plate | BSA | FBG | TFN | PrA | PrG |
|---|---|---|---|---|---|---|
| 1.5 | SIO | 101 | 51 | 64 | 99 | 100 |
| 3 | SIO | 99 | 63 | 62 | 99 | 102 |
| 1.5 | CA | 84 | 76 | 44 | 38 | 44 |
| 3 | CA | 81 | 83 | 46 | 63 | 52 |

**Example 10**

A test similar to Example 7 was carried out to compare 384 Well 55 μL (SiO) vs 200 μL (CA) shallow plates. The conditions and results are shown in Table 8. For the BSA, PrA, and PrG proteins, the SIO plates provided substantially superior protein recovery, compared to the CA plates. The FBG proteins did not demonstrate substantially superior protein recovery.

**Table 9**
**RECOVERY@72 hrs in** % (384 Well 55 μL (SiO) or 200 μL (CA) Shallow Plate)

| Concentration (nM) | Plate | BSA | FBG | TFN | PrA | PrG |
|---|---|---|---|---|---|---|
| 1.5 | SIO | 97 | 38 | 92 | 71 | 102 |

(continued)

| RECOVERY@72 hrs in % (384 Well 55 µL (SiO) or 200 µL (CA) Shallow Plate) | | | | | | |
| Concentration (nM) | Plate | BSA | FBG | TFN | PrA | PrG |
|---|---|---|---|---|---|---|
| 3 | SIO | 102 | 57 | 87 | 92 | 104 |
| 1.5 | CA | 34 | 58 | 32 | 27 | 14 |
| 3 | CA | 63 | 62 | 39 | 37 | 28 |

## Example 11

A test similar to Example 1 was carried out to compare the SIO treated plates to polypropylene plates treated with StabilBlot® BSA Blocker, a commercial treatment used to reduce BSA protein adhesion, sold by SurModics, Inc., Eden Prairie, MN, USA. The conditions and results are shown in Table 10, where SIO is the plate according to Example 1, Plate A is a polypropylene plate treated with 5% BSA blocker for one hour and Plate B is a polypropylene plate treated with 1% BSA blocker for one hour. Except for FBG protein, the present invention again provided superior results compared to the BSA blocker plates.

**Table 10**
**RECOVERY@24 hrs in % (3 nM in buffer)**

| Concentration (nM) | Plate | BSA | FBG | TFN | PrA | PrG |
|---|---|---|---|---|---|---|
| 3 | SIO | 102 | 69 | 79 | 96 | 104 |
| 3 | A | 97 | 85 | 71 | 93 | 102 |
| 3 | B | 94 | 84 | 66 | 70 | 75 |

A: 5% BSA blocker passivation of 1000 µL, treated 1hr;
B: 1% BSA blocker passivation of 1000 µL, treated 1hr;

## Example 12

A test similar to Example 7 was carried out to compare the protein recovery rates of SIO treated plates in accordance with Example 1 over longer periods of time - from 1 to 4 months. The conditions and results are shown in Table 12, which illustrates that roughly uniform resistance to protein adhesion was observed for all of the proteins over a substantial period.

**Table 12**
**RECOVERY in %**

| Time (month) | BSA | FBG | TFN | PrA | PrG |
|---|---|---|---|---|---|
| Initial | 97 | 80 | 63 | 98 | 101 |
| 1 | 95 | - | 66 | 81 | 100 |
| 2 | 94 | 85 | 61 | 87 | 96 |
| 3 | 92 | 77 | 79 | 87 | 94 |
| 4 | 97 | 74 | 77 | 85 | 94 |

## Example 13

The uniformity of binding among the different wells of a single plate was tested using two 96-well plates with deep (500 µL) wells, one and SIO plate prepared according to Example 1 except testing 2nM PrA protein after two hours in all 96 wells, and the other a Competitor A plate, again testing 2nM PrA protein after two hours in all 96 wells. The protein recovery from each well on one plate was measured, then averaged, ranged (determining the highest and lowest recovery rates among the 96 wells), and a standard deviation was calculated. For the SIO plate, the mean recovery was 95%, the range of recoveries was 11%, and the standard deviation was 2%. For the CA plate, the mean recovery was 64%, the range of recoveries was 14%, and the standard deviation was 3%.

The same test as in the preceding paragraph was also carried out using 96-well plates with 1000 µL wells. For the SIO plate, the mean recovery was 100%, the range of recoveries was 13%, and the standard deviation was 3%. For the CA plate, the mean recovery was 62%, the range of recoveries was 25%, and the standard deviation was 3%.

This testing indicated that the SIO treatment of Example 1 allows at least as uniform a recovery rate among the different wells as the protein resisting coating of the CA plate. This suggests that the SIO plasma treatment is very uniform across the plate.

**Claims**

1. A method for treating a surface of a plastic substrate, comprising: treating the surface to form a converted surface, in which treating the surface is carried out by:

   employing a first treatment step that includes remote conditioning treatment of the surface with conditioning plasma of one or more non-polymerizing compounds at a remote point, where the ratio of the radiant energy density at the remote point to the radiant energy density at the brightest point of the conditioning plasma is less than 0.5, alternatively less than 0.25, alternatively substantially zero, alternatively zero; and
   employing a second treatment step that includes remote conversion treatment of the surface with a conversion plasma of water vapor to form a converted surface, where the ratio of the radiant energy density at the remote point of conversion treatment to the radiant energy density at the brightest point of the conversion plasma is less than 0.5, alternatively less than 0.25, alternatively substantially zero, alternatively zero, wherein the radiant energy density at the brightest point of the plasma is determined spectrophotometrically by measuring the radiant intensity of the most intense emission line of light in the spectrum from 380 nm to 750 nm wavelength at the brightest point, and the radiant energy density at the remote point is determined spectrophotometrically by measuring the radiant energy density of the same emission line of light at the remote point;
   wherein after the second treatment step the converted surface has a biomolecule recovery percentage greater than the biomolecule recovery percentage of the surface prior to treatment according to the method, wherein the biomolecule recovery percentage is determined according to the method "Testing of all embodiments" described in the description.

2. The method of claim 1, wherein the surface is a vessel lumen surface.

3. The method of any one preceding claim, wherein the plastic substrate comprises olefin polymer, polypropylene (PP), polyethylene (PE), cyclic olefin copolymer (COC), cyclic olefin polymer (COP), polymethylpentene, polyester, polyethylene terephthalate, polyethylene naphthalate, polybutylene terephthalate (PBT), polyvinylidene chloride (PVdC), polyvinyl chloride (PVC), polycarbonate, polylactic acid, polystyrene, hydrogenated polystyrene, polycyclohexylethylene (PCHE), epoxy resin, nylon, polyurethane polyacrylonitrile, polyacrylonitrile (PAN), an ionomeric resin, or any combination, composite or blend of any two or more of the above materials.

4. The method of any one preceding claim, in which the surface is a coating or layer of PECVD deposited $SiO_xC_yH_z$ or $SiN_xC_yH_z$, in which x is from about 0.5 to about 2.4 as measured by X-ray photoelectron spectroscopy (XPS), y is from about 0.6 to about 3 as measured by XPS, and z is from about 2 to about 9 as measured by Rutherford backscattering spectrometry (RBS); or a barrier coating or layer of $SiO_x$, in which x is from about 1.5 to about 2.9 as measured by XPS, alternatively an oxide or nitride of an organometallic precursor that is a compound of a metal element from Group III and/or Group IV of the Periodic Table.

5. The method of any one preceding claim, wherein the surface of the substrate is a fluid contact surface of a microplate, a centrifuge tube, a pipette tip, a well plate, a microwell plate, an ELISA plate, a microtiter plate, a 96-well plate, a 384-well plate, a vial, a bottle, a jar, a syringe, a cartridge, a blister package, an ampoule, an evacuated blood collection tube, a specimen tube, a centrifuge tube, or a chromatography vial.

6. The method of any one preceding claim, wherein the non-polymerizing compound comprises, consists essentially of or consists of one or more of a member selected from the group consisting of: $O_2$, $N_2$, air, $O_3$, $N_2O$, $H_2$, $H_2O_2$, $NH_3$, Ar, He and Ne.

7. The method of claim 6, wherein the non-polymerizing compound comprises, consists essentially of or consists of $O_2$ and/or $N_2$.

8. The method of claim 7, wherein the non-polymerizing compound in the first treatment step consists of $N_2$.

9. The method of any one preceding claim, wherein the first treatment step includes one or more of the following process parameters: power of 50-600 W, gas flow rate of 5-50 sccm, time of 0.5-5 minutes and pressure of 0-1,000 mTorr; and the second treatment step includes one or more of the following process parameters: power of 50-600 W, gas flow rate of 1-10 sccm, time of 0.5-5 minutes and pressure of 0-1,000 mTorr.

10. An article containing a plastic substrate with a surface treated according to a method as described in any one preceding claim.


**Patentansprüche**

1. Verfahren zur Behandlung einer Oberfläche eines Kunststoffsubstrats, umfassend:

   Behandeln der Oberfläche, um eine umgewandelte Oberfläche zu bilden, wobei Behandeln der Oberfläche durchgeführt wird durch:

   Verwenden eines ersten Behandlungsschritts, der Fernkonditionierungsbehandlung der Oberfläche mit Konditionierungsplasma von einer oder mehreren nichtpolymerisierenden Verbindungen an einem entfernten Punkt einschließt, wobei das Verhältnis der Strahlungsenergiedichte an dem entfernten Punkt zu der Strahlungsenergiedichte an dem hellsten Punkt des Konditionierungsplasmas weniger als 0,5, alternativ weniger als 0,25, alternativ im Wesentlichen Null, alternativ Null beträgt; und
   Verwenden eines zweiten Behandlungsschritts, der Fernumwandlungsbehandlung der Oberfläche mit einem Umwandlungsplasma aus Wasserdampf einschließt, um eine umgewandelte Oberfläche zu bilden, wobei das Verhältnis der Strahlungsenergiedichte an dem entfernten Punkt der Umwandlungsbehandlung zu der Strahlungsenergiedichte an dem hellsten Punkt des Umwandlungsplasmas weniger als 0,5, alternativ weniger als 0,25, alternativ im Wesentlichen Null, alternativ Null beträgt, wobei die Strahlungsenergiedichte an dem hellsten Punkt des Plasmas spektrophotometrisch bestimmt wird, indem die Strahlungsintensität der intensivsten Emissionslinie des Lichts in dem Spektrum von 380 nm bis 750 nm Wellenlänge am hellsten Punkt gemessen wird, und die Strahlungsenergiedichte an dem entfernten Punkt spektrophotometrisch bestimmt wird, indem die Strahlungsenergiedichte derselben Emissionslinie des Lichts an dem entfernten Punkt gemessen wird;

   wobei die umgewandelte Oberfläche nach dem zweiten Behandlungsschritt eine prozentuale Biomolekülwiederfindung hat, die größer als die prozentuale Biomolekülwiederfidung der Oberfläche vor der Behandlung gemäß dem Verfahren ist, wobei die prozentuale Biomolekülwiederfindung gemäß dem Verfahren "Testen aller Ausführungsformen" bestimmt wird, das in technischen Angaben beschrieben ist.

2. Verfahren nach Anspruch 1, wobei die Oberfläche eine Gefäßlumenoberfläche ist.

3. Verfahren nach einem der vorhergehenden Ansprüche, wobei das Kunststoffsubstrat Olefinpolymer, Polypropylen (PP), Polyethylen (PE), cyclisches Olefincopolymer (COC), cyclisches Olefinpolymer (COP), Polymethylpenten, Polyester, Polyethylenterephthalat, Polyethylennaphthalat, Polybutylenterephthalat (PBT), Polyvinylidenchloride (PVdC), Polyvinylchlorid (PVC), Polycarbonat, Polymilchsäure, Polystyrol, hydriertes Polystyrol, Polycyclohexylethylen (PCHE), Epoxyharz, Nylon, Polyurethan-Polyacrylnitril, Polyacrylnitril (PAN), ein Ionomerharz oder jegliche Kombination, jegliches Verbundmaterial oder Gemisch aus jedweden zwei oder mehr der obigen Materialien umfasst.

4. Verfahren nach einem der vorhergehenden Ansprüche, wobei die Oberfläche eine Beschichtung oder Schicht aus PECVD-abgeschiedenem $SiO_xC_yH_z$ oder $SiN_xC_yH_z$, wobei x etwa 0,5 bis etwa 2,4 ist, gemessen mittels Röntgenphotoelektronenspektroskopie (XPS), y etwa 0,6 bis etwa 3 ist, gemessen mittels XPS, und z etwa 2 bis etwa 9 ist, gemessen mittels Rutherford-Rückstreuungsspektrometrie (RBS); oder eine Barrierebeschichtung oder -schicht aus $SiO_x$, wobei x etwa 1,5 bis etwa 2,9 ist, gemessen mittels XPS, alternativ ein Oxid oder Nitrid eines organometallischen Vorläufers ist, der eine Verbindung eines Metallelements aus der Gruppe III und/oder IV des Periodensystems ist.

5. Verfahren nach einem der vorhergehenden Ansprüche, wobei die Oberfläche des Substrats eine Fluidkontaktoberfläche einer Mikroplatte, eines Zentrifugenröhrchens, einer Pipettenspitze, einer Well-Platte, einer Mikrowell-Platte, einer ELISA-Platte, einer Mikrotiterplatte, einer 96-Well-Platte, einer 384-Well-Platte, eines Vials, einer Flasche, eines Bechers, einer Spritze, einer Kartusche, einer Blisterpackung, einer Ampulle, eines evakuierten Blutabnahmeröhrchens, eines Probenröhrchens, eines Zentrifugenröhrchens oder eines Vials für die Chromatographie ist.

**6.** Verfahren nach einem der vorhergehenden Ansprüche, wobei die nichtpolymerisierende Verbindung ein oder mehrere von einem Element ausgewählt aus der Gruppe bestehend aus $O_2$, $N_2$, Luft, $O_3$, $N_2O$, $H_2$, $H_2O_2$, $NH_3$, Ar, He und Ne umfasst, im Wesentlichen daraus besteht oder daraus besteht.

**7.** Verfahren nach Anspruch 6, wobei die nichtpolymerisierende Verbindung $O_2$ und/oder $N_2$ umfasst, im Wesentlichen daraus besteht oder daraus besteht.

**8.** Verfahren nach Anspruch 7, wobei die nichtpolymerisierende Verbindung in dem ersten Behandlungsschritt aus $N_2$ besteht.

**9.** Verfahren nach einem der vorhergehenden Ansprüche, wobei der erste Behandlungsschritt ein oder mehrere der folgenden Prozessparameter einschließt: Leistung von 50-600 W, Gasflussrate von 5-50 Standard-cm$^3$/min, Zeit von 0,5-5 Minuten und Druck von 0-1000 mTorr; und der zweite Behandlungsschritt ein oder mehrere der folgenden Prozessparameter einschließt: Leistung von 50-600 W, Gasflussrate von 1-10 Standard-cm$^3$/min, Zeit von 0,5-5 Minuten und Druck von 0-1000 mTorr.

**10.** Artikel, der ein Kunststoffsubstrat mit einer Oberfläche enthält, die gemäß einem Verfahren wie in einem der vorhergehenden Ansprüche beschrieben behandelt worden ist.

## Revendications

**1.** Procédé pour le traitement d'une surface d'un substrat de matière plastique, comprenant : le traitement de la surface pour former une surface convertie, dans lequel le traitement de la surface est effectué en :

employant une première étape de traitement qui comprend un traitement de conditionnement à distance de la surface avec un plasma de conditionnement d'un ou plusieurs composés non polymérisants en un point à distance, où le rapport de la densité d'énergie radiante au niveau du point à distance sur la densité d'énergie radiante au niveau du point le plus brillant du plasma de conditionnement est inférieur à 0,5, en variante inférieur à 0,25, en variante sensiblement de zéro, en variante zéro ; et
employant une deuxième étape de traitement qui comprend un traitement de conversion à distance de la surface avec un plasma de conversion de vapeur d'eau pour former une surface convertie, où le rapport de la densité d'énergie radiante au niveau du point à distance du traitement de conversion sur la densité d'énergie radiante au niveau du point le plus brillant du plasma de conversion est inférieur à 0,5, en variante inférieur à 0,25, en variante sensiblement de zéro, en variante zéro, la densité d'énergie radiante au niveau du point le plus brillant du plasma étant déterminée de manière spectrophotométrique en mesurant l'intensité radiante de la ligne d'émission de lumière la plus intense dans le spectre de 380 nm à 750 nm de longueur d'onde au niveau du point le plus brillant, et la densité d'énergie radiante au niveau du point à distance étant déterminée de manière spectrophotométrique en mesurant la densité d'énergie radiante de la même ligne d'émission de lumière au niveau du point à distance ;
dans lequel après la deuxième étape de traitement, la surface convertie possède un pourcentage de récupération de biomolécules plus grand que le pourcentage de récupération de biomolécules de la surface avant le traitement selon le procédé, le pourcentage de récupération de biomolécules étant déterminé selon le procédé « test de tous les modes de réalisation » décrit dans la description.

**2.** Procédé selon la revendication 1, la surface étant une surface de lumière de cuve.

**3.** Procédé selon l'une quelconque des revendications précédentes, le substrat de matière plastique comprenant un polymère d'oléfine, un polypropylène (PP), un polyéthylène (PE), un copolymère d'oléfine cyclique (COC), un polymère d'oléfine cyclique (COP), un polyméthylpentène, un polyester, un poly(téréphtalate d'éthylène), un poly(naphtalate d'éthylène), poly(téréphtalate de butylène) (PBT), un poly(chlorure de vinylidène) (PVdC), un poly(chlorure de vinyle) (PVC), un polycarbonate, un poly(acide lactique), un polystyrène, un polystyrène hydrogéné, un polycyclohexyléthylène (PCHE), une résine époxy, un nylon, un polyuréthane polyacrylonitrile, un polyacrylonitrile (PAN), une résine ionomère ou une quelconque combinaison, un quelconque composite ou mélange de deux quelconques ou plus des matériaux ci-dessus.

**4.** Procédé selon l'une quelconque des revendications précédentes, dans lequel la surface est un revêtement ou une couche de $SiO_xC_yH_z$ ou $SiN_xC_yH_z$ déposé(e) par PECVD, dans lequel/laquelle x est d'environ 0,5 à environ 2,4 tel que mesuré par spectroscopie photoélectronique à rayons X (XPS), y est d'environ 0,6 à environ 3 tel que mesuré par XPS, et z est d'environ 2 à environ 9 tel que mesuré par spectrométrie de rétrodiffusion de Rutherford (RBS) ; ou un revêtement ou une couche barrière de $SiO_x$, dans lequel/laquelle x est d'environ 1,5 à environ 2,9 tel que mesuré par XPS, en variante un oxyde ou nitrure d'un précurseur organométallique qui est un composé d'un élément métallique du groupe III et/ou du groupe IV du tableau périodique.

5. Procédé selon l'une quelconque des revendications précédentes, la surface du substrat étant une surface de contact de fluide d'une microplaque, un tube de centrifugation, un embout de pipette, une plaque à puits, une plaque à micropuits, une plaque ELISA, une plaque de microtitration, une plaque à 96 puits, une plaque à 384 puits, un flacon, une bouteille, un pot, une seringue, une cartouche, un emballage-coque, une ampoule, un tube de prélèvement sanguin sous vide, un tube d'échantillon, un tube de centrifugation ou un flacon de chromatographie.

6. Procédé selon l'une quelconque des revendications précédentes, le composé non polymérisant comprenant, étant essentiellement constitué de ou étant constitué d'un ou plusieurs parmi un membre choisi dans le groupe constitué par : $O_2$, $N_2$, air, $O_3$, $N_2O$, $H_2$, $H_2O_2$, $NH_3$, Ar, He et Ne.

7. Procédé selon la revendication 6, le composé non polymérisant comprenant, étant essentiellement constitué de ou étant constitué de $O_2$ et/ou $N_2$.

8. Procédé selon la revendication 7, le composé non polymérisant dans la première étape de traitement étant constitué de $N_2$.

9. Procédé selon l'une quelconque des revendications précédentes, la première étape de traitement comprenant l'un ou plusieurs des paramètres de processus suivants : puissance de 50 à 600 W, débit de gaz de 5 à 50 sccm, durée de 0,5 à 5 minutes et pression de 0 à 1 000 mTorr ; et la deuxième étape de traitement comprenant l'un ou plusieurs des paramètres de processus suivants : puissance de 50 à 600 W, débit de gaz de 1 à 10 sccm, durée de 0,5 à 5 minutes et pression de 0 à 1 000 mTorr.

10. Article contenant un substrat de matière plastique doté d'une surface traitée selon un procédé tel que décrit dans l'une quelconque des revendications précédentes.

FIG. 1

FIG. 2

FIG. 3

% Protein Recovery
at 24 Hrs

90%

72%

Remote Plasma    Direct Plasma

FIG. 4

% Protein Recovery
at 24 Hrs

# FIG. 5

FIG. 6

**FIG. 7**

FIG. 8

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7985188 B

- WO 2013071138 A

**Non-patent literature cited in the description**

- Pulsed Plasma-Pulsed Injection Sources For Remote Plasma Activated Chemical Vapor Deposition. **MARK J. KUSHNER.** J. APPL. PHYS. 1993, vol. 73, 4098